(19) Europäisches Patentamt
European Patent Office
Office européen des brevets

(11) EP 2 689 032 B1

(12) EUROPEAN PATENT SPECIFICATION

(45) Date of publication and mention of the grant of the patent:
**30.05.2018 Bulletin 2018/22**

(21) Application number: **12714582.9**

(22) Date of filing: **21.03.2012**

(51) Int Cl.:
***C12Q 1/68*** *(2018.01)*

(86) International application number:
**PCT/EP2012/001602**

(87) International publication number:
**WO 2012/126639 (27.09.2012 Gazette 2012/39)**

# (54) MODIFIED HYBRIDIZATION PROBES

MODIFIZIERTE HYBRIDISIERUNGSSONDEN

SONDES D'HYBRIDATION MODIFIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.03.2011 EP 11159652**

(43) Date of publication of application:
**29.01.2014 Bulletin 2014/05**

(73) Proprietor: **Qiagen GmbH**
**40724 Hilden (DE)**

(72) Inventors:
- **ENGEL, Holger**
  **40724 Hilden (DE)**
- **AZZAWI, Alexander**
  **40724 Hilden (DE)**
- **DI PASQUALE, Francesca**
  **40724 Hilden (DE)**

(74) Representative: **CH Kilger Anwaltspartnerschaft mbB**
**Fasanenstrasse 29**
**10719 Berlin (DE)**

(56) References cited:
**WO-A1-2010/058189**
**WO-A1-2010/067874**
**WO-A2-2004/111072**
**WO-A2-2009/045067**
**US-A1- 2002 001 844**
**US-A1- 2003 170 711**
**US-A1- 2006 014 189**
**US-A1- 2007 212 695**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 689 032 B1

SEQUENCE LISTING

<110> QIAGEN GmbH

<120> MODIFIED HYBRIDIZATION PROBES

<130> B051-0079WO1

<150> EP 11 15 9652.4
<151> 2011-03-24

<160> 14

<170> PatentIn version 3.5

<210> 1
<211> 25
<212> DNA
<213> Artificial Sequence

<220>
<223> probe/primer

<400> 1
agcaattgcg ggacgggacc gacta 25

<210> 2
<211> 25
<212> DNA
<213> Artificial Sequence

<220>
<223> probe/primer

<400> 2
agcaattgcg ggatgggacc gacta 25

<210> 3
<211> 19
<212> DNA
<213> Artificial Sequence

<220>
<223> primer/probe

<400> 3
tcggtcccgt cccgcaatt 19

<210> 4
<211> 18
<212> DNA
<213> Artificial Sequence

<220>
<223> primer/probe

<220>
<221> misc_structure
<222> (9)..(9)
<223> Abasic site

<400> 4
tcggtccctc ccgcaatt 18

<210> 5
<211> 18
<212> DNA
<213> Artificial Sequence

<220>
<223> primer/probes

<220>
<221> misc_structure
<222> (9)..(9)
<223> /ispc3/ abasic site at position 9;

<400> 5
tcggtccctc ccgcaatt 18

<210> 6
<211> 20
<212> DNA
<213> Artificial Sequence

<220>
<223> primer/probe

<400> 6

gtgaagagcc scgtgtgctc 20

<210> 7
<211> 20
<212> DNA
<213> Artificial Sequence

<220>
<223> primer/probe

<400> 7
tatatattgt caccataagc 20

<210> 8
<211> 20
<212> DNA
<213> Artificial Sequence

<220>
<223> probe/primer

<400> 8
cactaccttc tgcagctatg 20

<210> 9
<211> 20
<212> DNA
<213> Artificial Sequence

<220>
<223> primer/probe

<400> 9
tctactctat ctatgctggc 20

<210> 10
<211> 29
<212> DNA
<213> Artificial Sequence

<220>
<223> primer/probe

<400> 10
taatcacatc aaactagcca tgcattcac 29

<210> 11
<211> 29
<212> DNA
<213> Artificial Sequence

<220>
<223> primer/probe

<400> 11
taatcacatc aaattagcca tgcattcac 29

<210> 12

<211> 28
<212> DNA
<213> Artificial Sequence

<220>
<223> primer/probe

<220>
<221> misc_structure
<222> (13)..(13)
<223> [spacer 3] at position 13

<400> 12
taatcacatc aaatagccat gcattcac 28

<210> 13
<211> 33
<212> DNA
<213> Artificial Sequence

<220>
<223> primerprobe

<400> 13
taatcacatc aaattagcca tgcattcacc aga 33

<210> 14
<211> 32
<212> DNA
<213> Artificial Sequence

<220>
<223> primer/probe

<220>
<221> misc_structure
<222> (13)..(13)
<223> [spacer 3] abasic site at position 13

<400> 14
taatcacatc aaatagccat gcattcacca ga 32

## Description

### Field of the Invention

The present invention is in the field of biology and diagnostics. It is more particularly in the field of molecular biology and in vitro diagnostics. More specifically it is in the field of nucleic acid amplification and multiplex amplification of preferentially RNA and DNA molecules.

### Background

It is generally known that nucleic acids which encode proteins are not always 100 % preserved. Mutations and SNPs (single nucleotide polymorphisms) are very common in each type of organism. Viruses are a particular example of this variability.

For the detection of the presence of an organism in a sample, PCR is often used for the amplification of the corresponding nucleic acids. One example thereof is the detection of pathogens in animal or human samples. There are various real-time PCR-based methods for this use. One of them is based on the melting profile analysis subsequent to amplification of the target. This can be achieved by means of intercalating dyes (like SybrGreen) and by using sequence-specific probes (such as e.g. TaqMan probes) which hybridise to the target nucleic acid. The length of the probe used for this purpose affects its specific melting temperature.

The multiplex real-time technology (WO 2009/135832) is a particular case. In case of a positive signal for a probe reporter dye, the origin of the signal, e.g. the identity of a pathogen, can be determined by combinatory classification of the melting temperature of the generated PCR product and the probe reporter dye.

For successful classification of the melting profile, the melting temperature of the generated PCR product must not vary outside measuring inaccuracy. The presence of sequence variations such as e.g. often with viral, microbial or other target sequences with variable genotypes within the probe-specific target sequence, would usually lead to a shift of the melting point since the probe cannot hybridise to the target in a perfect way. Often, this problem cannot be solved by changing the position of the probe to another region, since the corresponding perfectly preserved regions are not present.

Numerous attempts have been made in order to address the problem of sequence variation in hybridization experiments.

Thus, the aim of the present invention is to provide for a method that allows amplification of potentially multiple nucleic acids, wherein at least one of said target nucleic acids displays sequence variation in a given population of a specific organism.

Abasic sites have been mentioned in "Compositions and Methods for Enhancing Hybridization and Priming Specificity" (US-B1 6,361,940) as well as in "Method for Label-free Detection of Hybridized DNA Target" (US-B1 6,579,680) and "Use of Abasic Site-Containing DNA Strands for Nucleobase Recognition in Water" Yoshimoto et al., JACS Communications, published on Web, 07/04/2003.

TMAC (tetramethylammonium chloride)

At high concentrations (3M), TMAC affects the melting temperature of DNA so that it is independent from the sequence but only dependent from the length of the oligonucleotide. TMAC 3M, however, is PCR inhibitory and, thus, cannot be used directly in a PCR reaction; see also "Alteration of the Relative Stability of dA·dT and dG·dC Base Pairs in DNA (Melchior and von Hippel, Proc. Nat. Acad. Sci. USA, February 1973, Vol. 70, No. 2, pp. 298-302).

Low concentrations of tetramethylammonium chloride increase yield and specificity of PCR; see also (Chevet, Lemaitre and Katinka, Nucleic Acids Research 1995, Vol. 23, No. 16, pp. 3343-3344).

Inosine is known as a universal base. However, in the literature it is disputed that this base can be designated as such since it has been proven that there are clear differences in the stability with different opposing bases; see also "Nearest-neighbor thermodynamics of deoxyinosine pairs in DNA duplexes" (Watkins, Jr and SantaLucia, Jr, Nucleic Acids Research, 2005, Vol, 33, No. 19, pp. 6258-6267).

Nebularine also exhibits a bias so that the pairing does not take place with all the four natural bases with the same stability.

Nitroindole and nitropyrrole are known modifications which are called universal bases. The differences in the melting temperature in the presence of different opposite bases are at least 2 °C.

### Summary of the invention

The scope of the invention is defined by the appended claims. The inventors have astonishingly found that

probes with abasic sites may help in detecting nucleic acids in hybridization and/or hybridization and amplification methods.

The disclosure relates to a method for detecting a nucleic acid, comprising the steps of, (i) amplifying the nucleic acid to be detected, (ii) during or after amplification, hybridizing to said nucleic acid to be detected a first probe that comprises an abasic site (apurinic/apyrimidinic site) additionally optionally carrying a detectable label, (iii) wherein the position of the abasic site corresponds to a position in said nucleic acid to be detected, known to have a polymorphism, and wherein said nucleic acid is detected if hybridization occurs.

In the most general form probes that comprise an abasic site are nucleic acids or oligonucleotides in which one or more bases are missing, the backbone or an appropriate structure to replace the backbone of the nucleic acid or oligonucleotide however is present. At the site of the missing base no hydrogen bonding or other specific interaction can thus take place with a complementary strand.

The term 'meltbridge' may be used to indicate an abasic site. Herein, meltbridge and abasic site in a nucleic acid are used synonymously.

Under certain conditions, abasic sites may be recognized by endonucleases or other enzymatic repair systems resulting in the cleavage of the backbone, i.e. single-stand breaks. However, this is to be avoided in the context of the present invention. Hence, the 'abasic site' in the context of the present invention is preferably a stable 'abasic site', i.e. an abasic site at which the backbone may not be cleaved. This can for example be achieved by the use of 1', 2'-dideoxyribose nucleoside analogues or synthetic linker molecules that replace the sugar-phosphate backbone at the abasic site. The preferred stable abasic sites are described in the following.

An abasic site may for example be generated by the incorporation of abasic DNA nucleoside analogues, abasic RNA nucleoside analogues and abasic linker molecules that replace the natural backbone structure. A preferred stable abasic DNA nucleoside analogue is based on a 1', 2'-dideoxyribose molecule without a base attached to the C1 atom of the dideoxyribose. A preferred example of an abasic RNA analogue is a 1'deoxyribose without a base at the C1 atom. As is outlined above, instead of a base also a different chemical moiety can be bound to the C1 atom of the spacer which, however, is not capable of forming hydrogen bonds or another specific interaction with the corresponding base of the complementary target strand.

Stable abasic sites in certain embodiments may have the following formula:

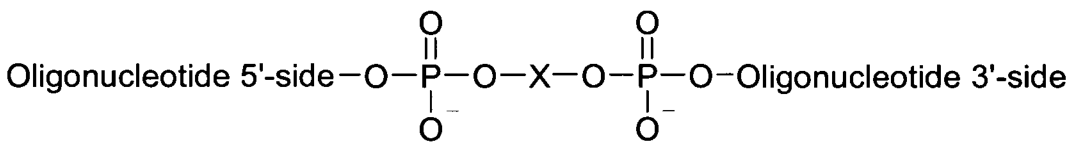

X may be selected from the following groups:

Group 1: X may be a branched or unbranched alkyl chain with 1 to 18 carbon atoms, preferably with 3 carbon atoms. The alkyl chain may comprise one or more heteroatoms such as S, O, N and P. It is preferred that X is derived from 1,3 propanediol (termed 'C3-linker' herein).

Group 2: X may be a polyethylenglycol chain with 1 to 20 glycol units.

Group 3: X may be derived from 1',2'-Dideoxyribose (termed 'dSpacer' herein) or 1'-Deoxyribose (termed 'rSpacer' herein) as shown in the following figures:

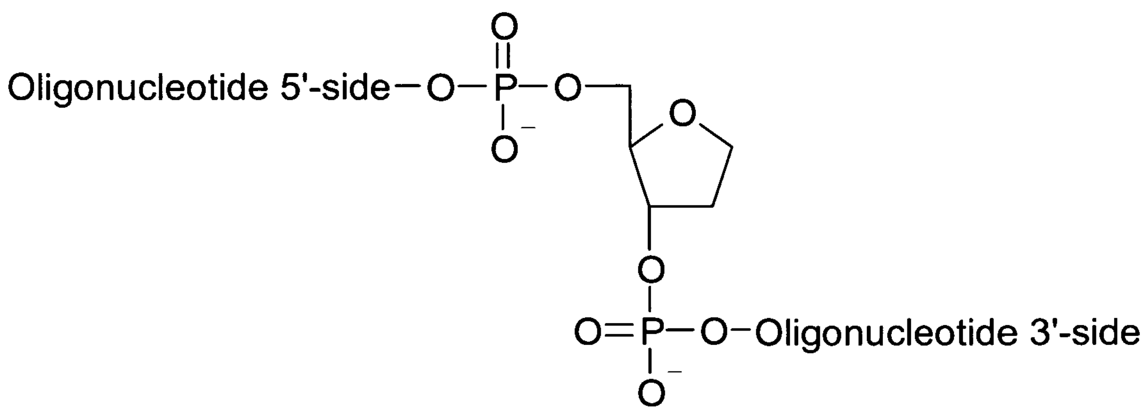

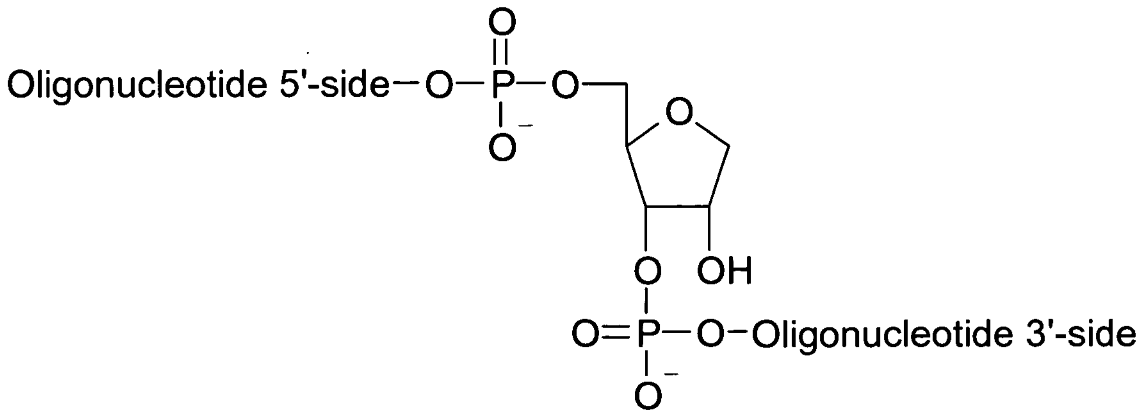

Other cyclic spacer molecules that generate a bridge of 2 to 6 C-atoms between the backbone phosphates of the probe resulting in stable abasic sites like cyclopentane and cyclohexane may also be used.

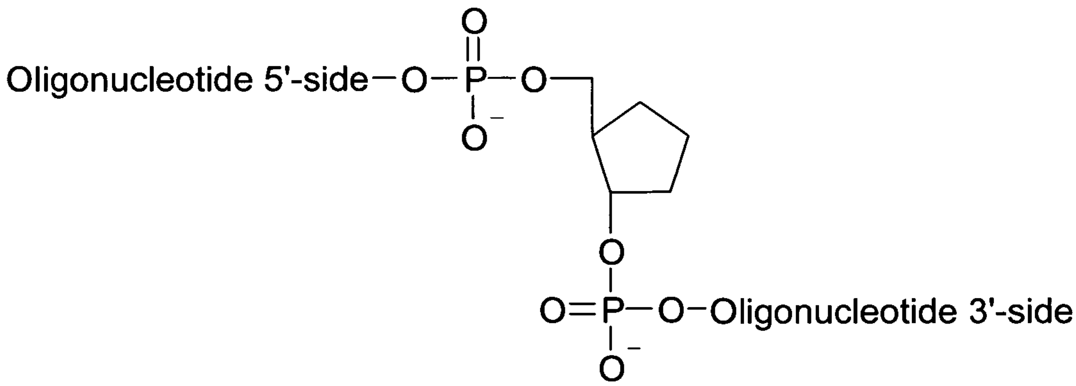

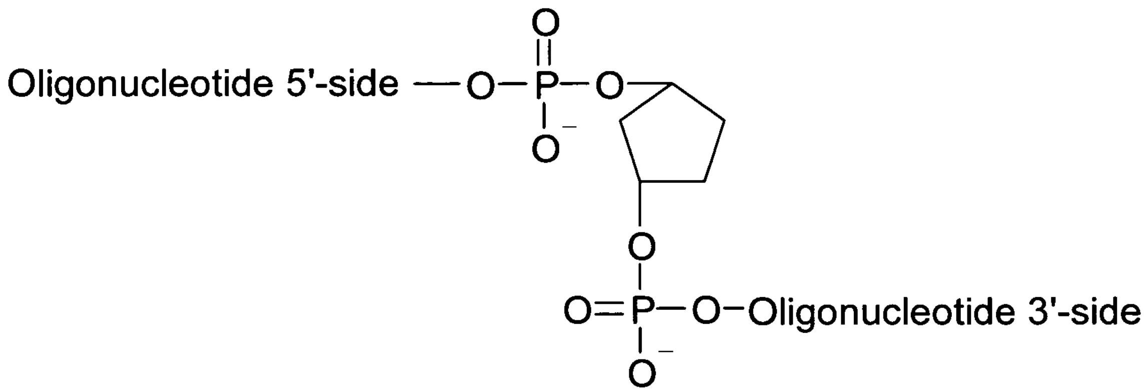

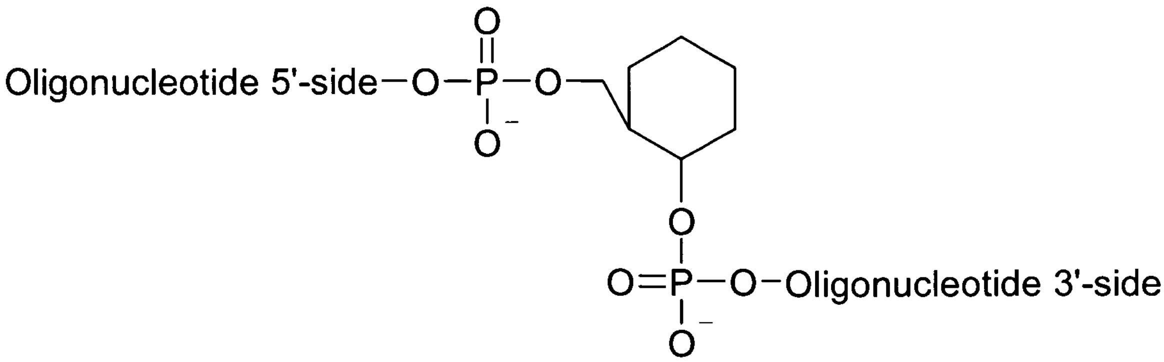

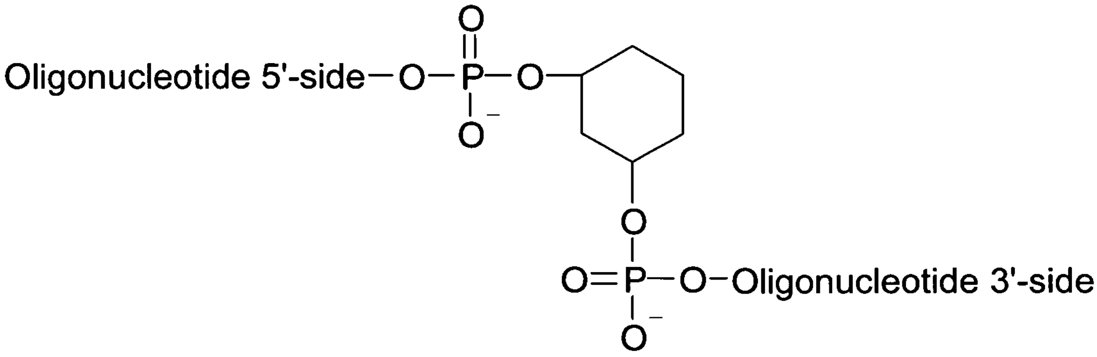

Hence, in principal the abasic sites in oligonucleotides or nucleic acid comprise at the 'abasic' position an abasic nucleoside or another chemical linker that does not form hydrogen bonds with the corresponding nucleobase in an opposing nucleic acid strand.

Solid-phase synthesis using the phosphoramidite method and phosphoramidite building blocks derived from protected 2'-deoxynucleosides (dA, dC, dG, and dT), ribonucleosides (A, C, G, and U), or chemically modified nucleosides, e.g. LNA is the preferred method for the synthesis of probes comprising an abasic site. To obtain the desired oligonucleotide, the building blocks are sequentially coupled to the growing oligonucleotide chain in the order required by the sequence of the product. Upon completion of the chain assembly, the product is released from the solid phase to solution, deprotected, and collected. The occurrence of side reactions sets practical limits for the length of synthetic oligonucleotides (up to about 200 nucleotide residues) because the number of errors accumulates with the length of the oligonucleotide being synthesized. Products are often isolated by HPLC to obtain the desired oligonucleotides in high purity. This synthesis is preferred also for oligonucleotides that contain abasic sites as claimed herein. A propyl linker may be incorporated with the phosphoramidite (3-(4,4'-dimethoxytrityloxy)propyl-1-[(2-cyanoethyl)-(N,N-diisopropyl)]-phosphoramidite).

Nucleosides are glycosylamines consisting of a nucleobase (often referred to as 'base') bound to a ribose or deoxyribose sugar via a beta-glycosidic linkage. The base is missing in nucleosides in abasic sites. A nucleotide is a nucleoside with one to three phosphate groups.

In one embodiment, the abasic nucleoside analogue is a compound having Formula I or II:

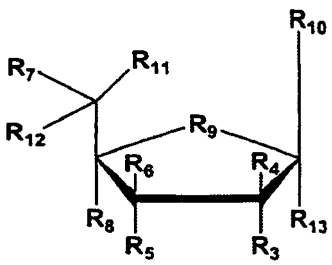

Formula I

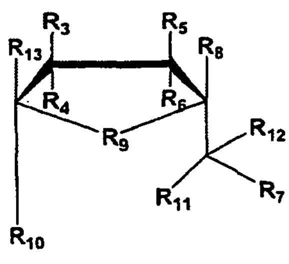

Formula II

wherein each R3, R4, R5, R6, R7, R8, R10, R11, R12, and R13 is independently H, OH, alkyl, substituted alkyl, alkaryl or aralkyl, F, Cl, Br, CN, CF3, OCF3, OCN, OPO3H2, O-alkyl, S-alkyl, N-alkyl, O-alkenyl, S-alkenyl, N-alkenyl, SO-alkyl, alkyl-SH, alkyl-OH, O-alkyl-OH, O-alkyl-SH, S-alkyl-OH, S-alkyl-SH, alkyl-S-alkyl, alkyl-O-alkyl, ONO2, NO2, N3, NH2, aminoalkyl, aminoacid, aminoacyl, ONH2, O-aminoalkyl, O-aminoacid, O-aminoacyl, heterocycloalkyl, heterocycloalkaryl, aminoalkylamino, polyalkylamino, or substituted silyl; R9 is O, S, CH2, S=O, CHF, or CF2.

Preferred examples of abasic chemical analogues (termed 'cSpacer' herein) that can be used in an oligonucleotide probe with an abasic site do not have a sugar backbone but are still capable of linking two ribose rings respectively deoxyribose rings of the antisense strand.

Several different abasic chemical analogues may be used. It is decisive that the formation of the duplex between probe and target nucleic acid is not disturbed by the abasic chemical. However, for this purpose, several abasic chemical analogues may be used. 1,3-propandiols based abasic sites and derivatives thereof are particularly suitable.

In one embodiment, the chemical abasic modification is a compound having Formula III:

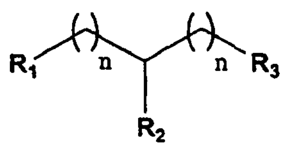

wherein each n is independently an integer from 0 to 12, each R1, R2 and R3 is independently H, OH, alkyl, substituted alkyl, alkaryl or aralkyl, F, Cl, Br, CN, CF3, OCF3, OCN, OPO3H2, O-alkyl, S-alkyl, N-alkyl, O-alkenyl, S-alkenyl, N-alkenyl, SO-alkyl, alkyl-SH, alkyl-OH, O-alkyl-OH, O-alkyl-SH, S-alkyl-OH, S-alkyl-SH, alkyl-S-alkyl, alkyl-O-alkyl, ONO2, NO2, N3, NH2, aminoalkyl, aminoacid, aminoacyl, ONH2, O-aminoalkyl, O-aminoacid, O-aminoacyl, heterocycloalkyl, heterocycloalkaryl, aminoalkylamino, polyalkylamino, or substituted silyl, and R1, and R3 serve as points of attachment to the neighbouring nucleotides of the siNA molecule of the invention. In a preferred embodiment, n is independently 0 or 1, in particular 1, R1 forms a phosphodiester linkage to the C5 position of the 3'-adjacent nucleotide, R3 forms a phosphodiester linkage to the C3 position of the 5'-adjacent nucleotide, and R2 is hydrogen or hydroxy, preferably hydrogen.

Preferred examples of analogues are described by the following formulae. The abasic modification can be accordingly derived there from.

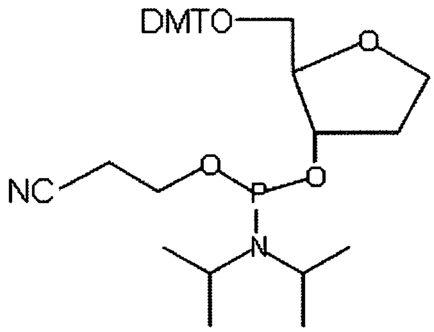

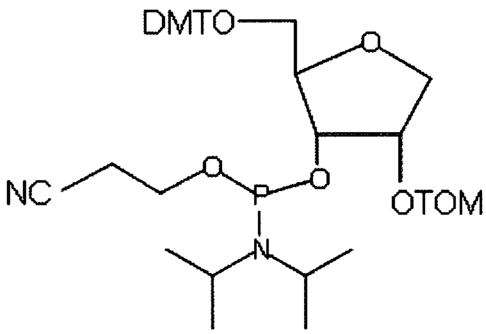

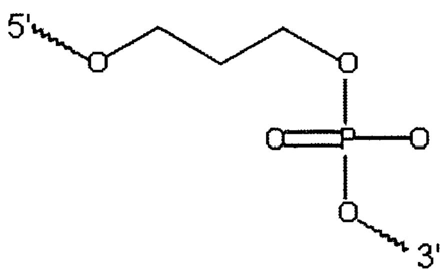

In the context of the present disclosure a nucleic acid may be, inter alia, RNA, DNA, cDNA (complementary DNA), LNA (locked nucleic acid), mRNA (messenger RNA), mtRNA (mitochondrial RNA), rRNA (ribosomal RNA), tRNA (transfer RNA), nRNA (nuclear RNA), siRNA (short interfering RNA), snRNA (small nuclear RNA), snoRNA (small nucleolar RNA), scaRNA (Small Cajal Body specific RNA), microRNA, dsRNA (doubled-stranded RNA), ribozyme, riboswitch, viral RNA, dsDNA (double-stranded DNA), ssDNA (single-stranded DNA), plasmid DNA, cosmid DNA, chromosomal DNA, viral DNA, mtDNA (mitochondrial DNA), nDNA (nuclear DNA), snDNA (small nuclear DNA) or the like or any other class or sub-class of nucleic acid which is distinguishable from the bulk nucleic acid in a sample.

In the context of the present disclosure fluorescent dyes may for example be FAM (5- or 6-carboxyfluorescein), VIC, NED, fluorescein, fluorescein isothiocyanate (FITC), IRD-700/800, cyanine dyes, such as CY3, CY5, CY3.5, CY5.5, Cy7, xanthen, 6-carboxy-2',4',7',4,7-hexachlorofluorescein (HEX), TET, 6-carboxy-4',5'-dichloro-2',7'-dimethoxyfluorescein (JOE), N,N,N',N'-tetramethyl-6-carboxyrhodamine (TAMRA), 6-carboxy-X-rhodamine (ROX), 5-carboxyrhodamine-6G (R6G5), 6-carboxyrhodamine-6G (RG6), rhodamine, rhodamine green, rhodamine red, rhodamine 110, BODIPY dyes, such as BODIPY TMR, Oregon Green, coumarines such as umbelliferone, benzimides, such as Hoechst 33258; phenanthridines, such as Texas Red, Yakima Yellow, Alexa Fluor, PET, ethidium bromide, acridinium dyes, carbazol dyes, phenoxazine dyes, porphyrine dyes, polymethin dyes, and the like, like as well as dyes with comparable fluorescent spectra and usability in the application.

In the context of the present disclosure amplification methods are for example, rolling circle amplification (such as in Liu, et al., "Rolling circle DNA synthesis: Small circular oligonucleotides as efficient templates for DNA polymerases," J. Am. Chem. Soc. 118:1587-1594 (1996)), isothermal amplification (such as in Walker, et al., "Strand displacement amplification an isothermal, in vitro DNA amplification technique," Nucleic Acids Res. 20(7):1691-6 (1992)), ligase chain reaction (such as in Landegren, et al., "A Ligase-Mediated Gene Detection Technique," Science 241:1077-1080, 1988, or, in Wiedmann, et al., "Ligase Chain Reaction (LCR)--Overview and Applications," PCR Methods and Applications (Cold Spring Harbor Laboratory Press, Cold Spring Harbor Laboratory, NY, 1994) pp. S51-S64)). Polymerase chain reaction amplification is preferred. Nucleic acid amplification can be accomplished by any of the various nucleic acid amplification methods known in the art, including but not limited to the polymerase chain reaction (PCR), real-time PCR (rtPCR), ligase chain reaction (LCR), transcription-based amplification system (TAS), nucleic acid sequence based amplification (NASBA), rolling circle amplification (RCA), transcription-mediated amplification (TMA), self-sustaining sequence replication (3SR) and Qβ amplification.

A possible detection method is the LUMINEX method.

Herein, a "label" is a moiety that is bound covalently or non-covalently to a probe where it can give rise to signal which may be detected by optical or other physical means.

Herein, a "polymorphism" is for example a single-nucleotide polymorphism (SNP) or mutation which is a DNA sequence variation occurring when a single nucleotide - A, T, C, or G - in the genome (or other shared sequence) differs between members of a species or paired chromosomes in an individual. For example, two sequenced DNA fragments from different individuals, AAGCCTA to AAGCTTA, contain a difference in a single nucleotide. In this case one says that there are two alleles: C and T.

Viruses undergo genetic change by several mechanisms. These include a process called genetic drift where individual bases in the DNA or RNA mutate to other bases. Most of these point mutations are "silent" - they do not change the protein that the gene encodes - but others can confer evolutionary advantages such as resistance to antiviral drugs. Antigenic shift occurs when there is a major change in the genome of the virus. This can be a result of recombination or reassortment. When this happens with influenza viruses, pandemics might result. RNA viruses often exist as quasi species or swarms of viruses of the same species but with slightly different genome nucleoside sequences. Such quasi species are a prime target for natural selection.

## Detailed description of the invention

The person skilled in the art will know that amplification requires reagents such, as for example, an enzyme for amplification, a buffer, nucleotides and the like. This of course depends on the type of amplification.

The inventors have developed a method which makes it possible to perform for example a multiplex amplification reaction with, for example 5 different targets, wherein probes with identical labels are used. The stable abasic site in the probe makes it possible to distinguish such targets, even if the template DNA comprises a SNP or a mutation. As can be seen from the experiments below, ordinarily, i.e. without abasic sites such probes would have a shift in melting temperature when hybridizing to and melting from a target with SNP or mutation. Such a shift would make it difficult to correctly detect the nucleic acid, as the probe would not display the expected melting temperature.

The melting temperature, Tm, of an oligonucleotide is its most critically important value. The most reliable and accurate determination of melting temperature is determined empirically.

Several useful, formulas have been developed to provide the Tm for PCR, Southern and Northern blots, and in situ hybridization.

The main factors affecting Tm are salt concentration, strand concentration, and the presence of denaturants (such as formamide or DMSO). Other effects such as sequence, length, and hybridization conditions can be important as well.

$T_m$ is the temperature at which 50 % of the oligonucleotide and its perfect complement are in duplex.

Td is the temperature at a particular salt concentration, and total strand concentration at which 50 % of an oligo and its perfect filter-bound complement are in duplex.

Equations

The simplest equation for Td is the Wallace rule:

$$(1)\ Td = 2\ °C\ (A+T) + 4\ °C\ (G+C)$$

Td is a filter-based calculation where A, G, C, and T are the number of occurrences of each nucleotide. This equation was developed for short DNA oligos of 14-20 base pairs hybridizing to membrane bound DNA targets in 0.9M NaCl.
The melting temperature for the sequence TGCTCA is, 2(1+2) + 4(1+2) = 18 °C. The nature of the immobilized target strand provides a net decrease in the Tm observed when both target and probe are free in solution. The magnitude of the decrease is approximately 7-8 °C.

Another familiar equation for DNA which is valid for oligos longer than 50 nucleotides from pH 5 to 9 is:

$$Tm = 81.5 + 16.6 \log M + 41(XG+XC) - 500/L - 0.62F$$

Where M is the molar concentration of monovalent cations, XG and XC are the mole fractions of G and C in the oligo, L is the length of the shortest strand in the duplex, and F is the molar concentration of formamide.

This is a far more useful equation to most researchers, as it includes adjustments for salt (although the equation is < when M = 0) and formamide, the two most common agents for changing hybridization temperatures.

Thus, at M = 0.9, and F = 0, Tm = 81.5+16.6 (log(0.9))+41(.17+.33)-500/6-0.62(0) = 17.9 ° C. Similar equations apply for RNA.

In one embodiment more than two different labels are used and all the probes that share a common label have a slightly varying melting temperature. All the probes that share a common melting temperature on the other hand have a different label. By detecting the label and the melting temperature either during or after amplification the inventors have for the first time provided for a means which makes it possible to analyze, e.g. said 20 templates in one tube even in the presence of target DNA or RNA which may or may not comprise one or more SNPs or mutations.

A probe as used herein is a nucleic acid molecule, such as, inter alia, RNA, DNA, cDNA (complementary DNA), LNA (locked nucleic acid), ssDNA (single-stranded DNA), or modified in the backbone, or the like which is capable of hybridizing to a further nucleic acid molecule. The probes may be over 1,000 nucleotides in length, are, however, preferably between 300 and 12 nucleotides, between 200 and 12 nucleotides, between 150 and 12 nucleotides, between 100 and 12 nucleotides, between 50 and 12 nucleotides or between 30 and 12 nucleotides in length. Shorter probes (about 100 to 12 nucleotides) are also referred to as oligonucleotides. Oligonucleotides may be prepared using any suitable method, such as, for example, the phosphotriester and phosphodiester methods or automated embodiments thereof. In one such automated embodiment diethyl phosphoramidites are used as starting materials and may be synthesized as described by Beaucage et al. (1981) Tetrahedron Letters 22:1859-1862. One method for synthesizing oligonucleotides on a modified solid support is described in US Patent No. 4,458,006.

The probe is labeled. Preferably, the label is a fluorescent dye. In a specific embodiment, the probe carries an additional quencher moiety that is able to quench the fluorescence of the label attached to the probe.

In one alternative, the melting temperature and/or point of said first hybridization probe is determined after hybridization. Methods where one measurement, i.e. melting point, is determined, are known such as melt analysis, HRM analysis or end-point analysis.

High-resolution melting analysis (HRM) is a closed-tube, post-PCR analysis that has raised enormous scientific interest. HRM characterizes double-stranded PCR products based on their dissociation (melting) behaviour. It is similar to the classical melting curve analysis, but provides far more information for a wider range of applications. PCR products can be discriminated according to sequence, length, GC content, or strand complementarity, down to single base-pair changes. Previously unknown and even complex sequence variations can be readily detected and characterized in a robust and straightforward way if probes are used that comprise an abasic site in case the target sequence comprises a SNP.

Also preferably, the method comprises at least one further probe which may optionally also comprise an abasic site but which carries the same label as said first hybridization probe and wherein the probes are distinguishable due to their difference in melting temperatures.

The abasic site may in a preferred embodiment be derived from 1'-2'-dideoxyribose or 1, 3-propandiol incorporated into the backbone of the probe.

Preferably in the method according to the invention (i) each of the probes is specific for a nucleic acid sequence, (ii) at least two probes carry the same label; and (iii) each of the probes that carry the same label has a melting temperature ($T_m$) which differs by more than 1 °C from the other probes.

It is preferred that the probes with the same label have melting temperatures that differ at least by 2 °C, preferably by at least about 5 °C, more preferably by between about 5 ° and 10 °C, even more preferably by between about 5 °C and 8 °C, even more preferably by between about 5 °C and 7 °C, even more preferably by between about 5 °C and 6 °C. Herein, in the context of temperature values, the term "about" is to be understood as to include deviations of up to +/- 10 % of the temperature value.

The amplification method is selected from the group of polymerase chain reaction (PCR), real-time PCR (rtPCR), ligase chain reaction (LCR), transcription-based amplification system (TAS), nucleic acid sequence based amplification (NASBA), rolling-circle amplification (RCA), transcription-mediated amplification (TMA), self-sustaining sequence replication (3SR) and Qβ amplification.

Preferably the amplification method is selected from the group of polymerase chain reaction (PCR) and real-time PCR (rtPCR) and the melting temperature and/or point of said probes is determined during PCR or post PCR.

A PCR reaction may consist of 10 to 100 "cycles" of denaturation and synthesis of a DNA molecule. In a preferred embodiment, the temperature at which denaturation is done in a thermocycling amplification reaction is between about 90 °C to greater than 95 °C, more preferably between 92-94 °C. Preferred thermo cycling amplification methods include polymerase chain reactions involving from about 10 to about 100 cycles, more preferably from about 25 to about 50 cycles, and peak temperatures from about 90 °C to greater than 95 °C, more preferably 92-94 °C. In a preferred embodiment, a PCR reaction is done using a DNA Polymerase I to produce, in exponential quantities relative to the number of reaction steps involved, at least one target nucleic acid sequence, given (a) that the ends of the target sequence are known in sufficient detail that oligonucleotide primers can be synthesized which will hybridize to them and (b) that a small amount of the target sequence is available to initiate the chain reaction. The product of the chain reaction will be a discrete nucleic acid duplex with termini corresponding to the ends of the specific primers employed.

Any source of nucleic acid, in purified or non-purified form, can be utilized as the starting nucleic acid, if it contains or is thought to contain the target nucleic acid sequence desired. Thus, the process may employ, for example, DNA which may be single-stranded or double-stranded. In addition, a DNA-RNA hybrid which contains one strand of each may be utilized. A mixture of any of these nucleic acids may also be employed, or the nucleic acids produced from a previous amplification reaction using the same or different primers may be so utilized. The nucleic acid amplified is preferably DNA or RNA or both. The target nucleic acid sequence to be amplified may be only a fraction of a larger molecule or can be present initially as a discrete molecule, so that the target sequence constitutes the entire nucleic acid. It is not necessary that the target sequence to be amplified be present initially in a pure form; it may be a minor fraction of a complex mixture or a portion of nucleic acid sequence due to a particular animal which organism might constitute only a very minor fraction of a particular biological sample. The starting nucleic acid may contain more than one desired target nucleic acid sequence which may be the same or different. Therefore, the method is useful for amplifying simultaneously multiple target nucleic acid sequences located on the same or different nucleic acid molecules. The nucleic acid(s) may be obtained from any source and include plasmids and cloned DNA, DNA from any source, including bacteria, yeast, viruses, and higher organisms such as plants or animals. DNA may be extracted from, for example, blood or other fluid, or tissue material such as chorionic villi or amniotic cells by a variety of techniques such as that described by Maniatis et al., Molecular Cloning: A Laboratory Manual, (New York: Cold Spring Harbor Laboratory) pp 280-281 (1982). Additionally the Templex technology may be applied which combines Genaco's Tem-PCR technology and Luminex' xMAP technology.

In one embodiment, in the PCR method, after hybridization the melting temperature and/or point of said first hybridization probe is determined.

Various kinds of labelled probes are known. These for example comprise a quencher and a label. Preferred are TaqMan probes, Scorpion probes, Molecular beacons, light cycler probes, LUX probes, amplifluor probes.

TaqMan probes are hydrolysis probes that are designed to increase the specificity of real-time PCR assays. The TaqMan probe principle relies on the 5'-3' nuclease activity of Taq polymerase to cleave a dual-labeled probe during hybridization to the complementary target sequence and fluorophore-based detection. As in other real-time PCR methods, the resulting fluorescence signal permits quantitative measurements of the accumulation of the product during the exponential stages of the PCR; however, the TaqMan probe significantly increases the specificity of the detection.

Like TaqMan probes, Molecular Beacons also use FRET (Fluorescene Resonance Energy Transfer) to detect and quantitate the synthesized PCR product via a fluorophore coupled to the 5' end and a quencher attached to the 3'

end of an oligonucleotide substrate. Unlike TaqMan probes, Molecular Beacons are designed to remain intact during the amplification reaction, and must re-bind to target in every cycle for signal measurement. Molecular Beacons form a stem-loop structure when free in solution. Thus, the close proximity of the fluorophore and quencher molecules prevents the probe from fluorescing. When a Molecular Beacon hybridizes to a target, the fluorescent dye and quencher are separated, FRET does not occur, and the fluorescent dye emits light upon irradiation.

With Scorpion probes, sequence-specific priming and PCR product detection is achieved using a single oligonucleotide. The Scorpion probe maintains a stem-loop configuration in the unhybridized state. The fluorophore is attached to the 5' end and is quenched by a moiety coupled to the 3' end. The 3' portion of the stem also contains a sequence that is complementary to the extension product of the primer. This sequence is linked to the 5' end of a specific primer via a non-amplifiable monomer. After extension of the Scorpion primer, the specific probe sequence is able to bind to its complement within the extended amplicon thus opening up the hairpin loop. This prevents the fluorescence from being quenched and a signal is observed.

A light cycler FRET probe system is a pair of single-stranded fluorescently-labeled oligonucleotides. Probe 1 (the donor probe) is labeled at its 3' end with a donor fluorophore (generally fluorescein) and Probe 2 (the acceptor probe) is labeled at its 5' end with one of four available fluorophores (red 610, 640, 670 or 705). The free 3'-hydroxyl group of Probe 2 must be blocked with a phosphate group (P) to prevent Taq DNA polymerase extension. To avoid any steric problems between the donor and the acceptor fluorophores on both probes, there should be a spacer of 1 to 5 nt (4 to 25 Å distance) to separate the two probes from each other. Before any real-time quantitative PCR reaction takes place, fluorescence background may be observed inside the tube.

During the annealing step of real-time quantitative PCR, the PCR primers and the light cycler probes hybridize to their specific target regions causing the donor dye to come into close proximity to the acceptor dye. When the donor dye is excited by light from the light cycler instrument (h$\gamma$1), energy is transferred by Fluorescence Resonance Energy Transfer (FRET) from the donor to the acceptor dye. The energy transfer causes the acceptor dye to emit light (h$\gamma$2) at a longer wavelength than the light emitted from the instrument (h$\gamma$1). The acceptor fluorophore's emission wavelength is detected by the instrument's optical unit. The increase in measured fluorescent signal is directly proportional to the amount of accumulating target DNA.

In one embodiment the melting transitions of the double-stranded segments can also be determined by monitoring fluorescence intensity of double-stranded nucleic acid-specific (dsNAS) dyes. In one embodiment, the double-stranded nucleic acid-specific dye is selected from the group consisting of SYBR® Green I, SYBR® Gold, ethidium bromide, propidium bromide, Pico Green, Hoechst 33258, YO-PRO-I and YO-YO-I, SYTO®9, LC Green®, LC Green® Plus+, EvaGreen™. These saturation dyes are capable of existing at sufficiently saturating conditions with respect to the DNA during or after amplification, while minimizing the inhibition of PCR. For example, at maximum PCR-compatible concentrations, the dsDNA binding dye has a percent saturation of at least 50 %. In other embodiments, the percent saturation is at least 80 %. In yet other embodiments, the percent saturation is at least 99 %. It is understood that the percent saturation is the percent fluorescence compared to fluorescence of the same dye at saturating concentrations. Saturating concentration is the concentration that provides the highest fluorescence intensity possible in the presence of a predetermined amount of dsDNA. Because these dyes can be present at significantly higher concentrations without significantly interfering with certain nucleic acid reactions, these dyes may be particularly useful for monitoring the conformation of single-stranded nucleic acids and dsDNA.

The invention relates also to a probe selected from the group of TaqMan probe, Scorpion probe, molecular beacon probe, light cycler probe, LUX probe and amplifluor probe, wherein the probe comprises at least one abasic site.

The disclosure also relates to a kit for the detection of a nucleic acid, wherein the kit comprises at least one probe with an abasic site. The probe in the kit is labelled.

Also, the probe in the kit with the abasic site may by a TaqMan probe, Scorpion probe, molecular beacon probe, light cycler probe, LUX probe or amplifluor probe.

Also, the kit may comprise two probes wherein these two carry the same label, one of which carries an abasic site and each of which are specific for a certain target.

**Figure captions**

**Fig. 1** illustrates the structure of the modifications (1', 2' dideoxyribose (dSpacer) and 1, 3-propanediol (C3-linker)).
**Fig. 2** illustrates the principle of the invention.
**Fig. 3** illustrates the protocol of the melting profile analysis.
**Fig. 4**
Melting profile analysis in the presence of the probe RhVrc-TM3 and the antisense oligonucleotides RhVrc AS3 (solid - perfect match) and RhVrc AS4 (dotted - a mismatch).

**Fig. 5**
Melting profile analysis in the presence of the probe RhVrc-TM7 (X=dSpacer) and the antisense oligonucleotides RhVrc AS3 (solid - perfect match) and RhVrc AS4 (dotted - a mismatch).
**Fig. 6**
Melting profile analysis in the presence of the probe RhVrc-TM8 (X= C3-linker) and the antisense oligonucleotides RhVrc AS3 (solid - perfect match) and RhVrc AS4 (dotted - a mismatch).
**Fig. 7**
Schematic representation of an alignment of sequences of two human Rhinovirus strains.
**Fig. 8**
The PCR protocol is represented in Figure 8 (5 min at 95 °C, followed by 50 cycles of 15 sec at 95 °C, 30 sec at 60 °C).
**Fig. 9**
Amplification plots and the subsequent melting profile analysis of a real-time PCR in the presence of an unmodified RhVrc-TM3 probe and different templates in different concentrations are shown. The solid curves show the reactions in the presence of the perfectly complementary template. The dotted curves show the reactions in the presence of an imperfectly complementary template.
**Fig. 10**
Amplification plots and the subsequent melting profile analysis of a real-time PCR in the presence of an RhVrc-TM7 probe modified with a stable abasic site and different templates in different concentrations are shown. The solid curves show the reactions in the presence of the perfectly complementary template. The dotted curves show the reactions in the presence of an imperfectly complementary template.
**Fig. 11**
Amplification plots and the subsequent melting profile analysis of a real-time PCR in the presence of an RhVrc-TM8 probe modified with a C3-linker and different templates in different concentrations are shown. The solid curves show the reactions in the presence of the perfectly complementary template. The dotted curves show the reactions in the presence of an imperfectly complementary template.

## EXAMPLES

The problem was solved by the use of modified probes carrying a meltbridge base (stable abasic site) at the position of the possible mutation (SNP). This meltbridge base is a so-called stable abasic site and can be both a 1',2'-dideoxyribose spacer (dSpacer) or a 1,3-propanediol linker (C3-linker); see also Fig. 1.

This meltbridge base has the particular advantage that it does not form a hydrogen bridge bond or otherwise specifically interact with the opposite base and thus, as shown in the following experiments, does not affect the melting point of the probe.
The modified probes have a defined melting temperature which is independent from the opposite base and can be measured in a narrow temperature range. The modified oligonucleotides were ordered and tested in various variants (different reporter dyes, different quenchers, different suppliers). A probe was used as control, which was not modified. All the reactions were carried out on a Rotor-Gene Q (QIAGEN, Hilden, Germany).

Herein, the following abbreviations are used, Tmelt or Tm (melting temperature), NTC (no template control) and IVT (in vitro transcript).

## Example 1

The melting temperature of the probes was first tested using artificial templates which were antisense oligonucleotides (oligonucleotides having the sequence which is reverse complementary to the probe and being longer than the probe due to 3 nucleotides both at the 5' and the 3' end). Two different antisense oligonucleotides were used, i.e. one with a perfectly complementary sequence to the control probe and the other one having a base substitution at the site directly complementary to the modification; see Table 1.

Table 1

| | | |
|---|---|---|
| SEQ ID NO. 1 | RhVrc AS3 | 5'AGCAATTGCGGGA**C**GGGACCGACTA-3' |
| SEQ ID NO. 2 | RhVrc AS4 | 5'AGCAATTGCGGGA**T**GGGACCGACTA-3' |

Table 1 shows the sequences of antisense oligonucleotides. The position where the antisense oligonucleotides differ from one another are marked, respectively. The sequence hybridizing to the probe used is underlined.

The sequences of the probes used are indicated in Table 2:

| | |
|---|---|
| RhVrc TM3 (SEQ ID NO. 3) | 5'/56 FAM/TCGGTCCC**G**TCCCGCAATT/3BHQ_1/3' |
| RhVrc TM7 (X=DSpacer) (SEQ ID NO. 4) | 5'/56 FAM/TCGGTCCC/**idSp**/TCCCGCAATT/3BHQ_1/3' |
| RhVrc TM8 (Y=C3-linker) SEQ ID NO. 5 | 5'/56 FAM/TCGGTCCC/**iSpC3**/TCCCGCAATT/3BHQ_1/3' |

Table 2 shows the sequences of the probes used. The position carrying the modification is marked in bold. The probes were ordered with the fluorophor FAM and with the quencher BHQ1.

Each one probe and an antisense oligonucleotide were added together as described below in Table 3.

Table 3:

| | Final concentration |
|---|---|
| Reaction Mix (2x) | 1x |
| Antisense oligonucleotide (10μM) | 0,3μM |
| Probe (10μM) | 0,3μM |
| H2O | ad 25μl |

Table 3 shows the composition of the mix for the melting profile analysis with antisense oligonucleotides.

Also, Fig. 3 illustrates the protocol for the melting profile analysis.

The melting profile analysis in the presence of the unmodified probe RhVrc-TM3 and the antisense oligonucleotide RhVrc AS3 (perfect match) lead to a melting peak at a temperature of 70.9 °C; see Fig. 4. In the same experiment, the melting profile was registered in the presence of the unmodified probe RhVrc-TM3 and the antisense oligonucleotide RhVrc AS4 (a mismatch). In this case, the melting temperature of the probe was 65 °C; see Fig. 4. The difference of 5.9 °C is due to the mismatch in the antisense oligonucleotide RhVrc AS4.

When the probes RhVrc-TM7 (X = dSpacer) and RhVrc-TM8 (Y = C3-linker) were used, the difference could be levelled in the melting temperature of the probes in the presence of perfect match or mismatch. Both antisense oligonucleotides RhVrc AS3 and RhVrc AS4 showed a comparable melting temperature in the presence of match and mismatch oligonucleotides; see Figs. 5 and 6.

A summary of the results is given in Table 4.

Table 4 (Melting temp. of probes used)

| Probe | Tmelt Perfect Match AS-Oligonucleotide RhVrc AS3 (°C) | Tmelt 1 Mismatch AS-Oligonucleotide RhVrc AS4 (°C) | Delta T (Match-Mismatch) (°C) |
|---|---|---|---|
| RhVrc-TM3 (unmodified) | 70.9 | 65 | 5.9 |
| RhVrc-TM7 (X= dSpacer) | 58 | 58.25 | 0.25 |
| RhVrc-TM8 (X= C3-linker) | 57.9 | 58.4 | 0.5 |

## Example 2

Example 2 demonstrates the feasibility of a PCR and melting profile analysis by means of modified probes which do not recognize differences between different mutations (FAM label).

This experiment is to show the feasibility of a real-time PCR reaction with subsequent melting profile analysis by means of probes modified by different spacers. We show, the melting temperature of the probe is not affected by the presence of templates with different sequences.

A segment of the genome of two variants of human Rhinovirus was used as an example. These were constructs which were to differ in the same base substitution as the antisense oligonucleotides of Example 1; see Fig. 7. These artificial sequences RhV2 and RhV4 were ordered as plasmid (Geneart AG).

The reactions were as shown in Table 5 (Composition of the PCR).

Table 5

| | Final Concentration |
|---|---|
| Reaction Mix (2x) | 1x |
| Primer RhV Fo (10µM) | 0,4µM |
| Primer RhV Ro (10µM) | 0,1µM |
| Probe (10µM) | 0,3µM |
| Template Plasmid | 500 - 50 - 5fg/RxN |
| H2O | ad 25µl |

For this purpose all primers indicated in Table 6 and probes indicated in Table 2 were used. All probes used were modified with the fluorescent dye FAM and BHQ1 quencher (IDT).

Table 6 shows the sequences of the primers used.

Table 6

| | |
|---|---|
| RhV Fo (SEQ ID NO. 6) | 5'-GTGAAGAGCCSCGTGTGCTC-3' |
| RhV Ro (SEQ ID NO. 7) | 5'-TATATATTGTCACCATAAGC-3' |

Control reactions were carried out in the presence of an unmodified probe (RhVrc-TM3). Reactions with template (solid and dotted curves in Fig. 9, Fig. 10 and Fig. 11) and without template (thick curves in Fig. 9, Fig. 10 and Fig. 11, water was added to all; no template was added) were carried out respectively. The reactions without template were carried out as control for possible occurrence of nonspecific background.

The PCR protocol is represented in Fig. 8 (5 min at 95 °C, followed by 50 cycles of 15 sec at 95 °C, 30 sec at 60 °C).

Fluorescence data (amplification plots) were registered during the 60 °C step. The PCRs were carried out using a Rotor Gene (Qiagen) with a reaction volume of 25 µl. Subsequently, the PCR products were subjected to a melting profile analysis.

Fig. 9, Fig. 10 and Fig. 11 show the amplification plots which were registered during the real-time PCR reaction in the presence of different probes and different templates. The curves show that all probes used can be used in real-time PCR.

Fig. 9, Fig. 10 and Fig. 11 show the melting profile analysis following the PCR. The unmodified probe RhVrc-TM3 showed different melting temperatures depending on which template, RhV2 or RhV4, was present in the reaction. The melting temperatures of the RhVrc-TM7 and the RhVrc-TM8 probes did not differ; in the presence of templates carrying a mutation, they did not differ either.

In all reactions, the NTCs did not show a peak. A summary of the melting temperatures is shown in Table 7.

Table 7 (Melting temperatures of the probes used)

| Probe | Tmelt RhV2 match (°C) | Tmelt RhV4 mismatch (°C) | Delta T (Match-Mismatch) (°C) |
|---|---|---|---|
| RhVrc-TM3 (unmodified) | 68 | 63,5 | 4,5 |
| RhVrc-TM7 (X=dSpacer) | 56,6 | 57 | 0,4 |
| RhVrc-TM8 (X= C3-linker) | 56,7 | 57,2 | 0,5 |

## Example 3

Example 3 demonstrates the feasibility of an RT-PCR and melting profile analysis by means of modified probes which do not recognize differences between different mutations (Quasar705 label).

The melting temperature of the probe is not affected by the presence of templates with different sequences. A segment of the genome of two variants of human parainfluenza virus 2 was used as an example. These were constructs which correspond, on the one hand, to human parainfluenza virus 2 (GenBank: NC_003443) and, on the other hand, to human parainfluenza virus strain V98 (GenBank: AF533011); see Fig. 12. In this segment, the two sequences selected differ by a base substitution, i.e. C to T. These artificial sequences were created as plasmids and transcribed into RNA

as in vitro transcript.

The reactions were mixed as indicated in Table 8.

Table 8 (RT-PCR mix)

| | Final Concentration |
|---|---|
| Reaction Mix (2x) | 1x |
| Primer PIV-2 Fi-ohne Tag (10μM) | 0,1μM |
| Primer PIV-2 Ri-ohne Tag (10μM) | 0,4μM |
| Probe (10μM) | 0,3μM |
| Template IVT | 10; 1fg/RxN |
| H2O | ad 25μl |

For this purpose, the primers indicated in Table 9 below and the probes indicated in Table 8 were used. The probe sequences differ in a single base substitution. All probes used are modified by means of the fluorescent dye Quasar705 and BHQ2 quencher (Biosearch Technologies). The dSpacer was not offered by this supplier, for this reason, only probes with the C3-linker were tested.

Table 9 (Sequence of the primers used)

| | |
|---|---|
| PIV-2 Fi-ohne Tag (SEQ ID NO. 8) | 5'-CACTACCTTCTGCAGCTATG-3' |
| PIV-2 Ri-ohne Tag (SEQ ID NO. 9) | 5'-TCTACTCTATCTATGCTGGC-3' |

Table 10 (Sequence of the probes used)

| | |
|---|---|
| Piv2-Probe1 (SEQ ID NO. 10) | 5'-Quasar705-TAATCACATCAAAcTAGCCATGCATTCAC-BHQ2-3' |
| Piv2-Probe2 (SEQ ID NO. 11) | 5'-Quasar705-TAATCACATCAAAtTAGCCATGCATTCAC-BHQ2-3' |
| PIV2 TM4 C3 (SEQ ID NO. 12) | 5'-Quasar705-TAATCACATCAAA[Spacer 3]TAGCCATGCATTCAC-BHQ2-3' |
| PIV2 TM6 (SEQ ID NO. 13) | 5'-Quasar705-TAATCACATCAAAtTAGCCATGCATTCACCAGA-BHQ2-3' |
| PIV2 TM7 C3 (SEQ ID NO. 14) | 5'-Quasar705-TAATCACATCAAA[Spacer 3]TAGCCATGCATTCACCAGA-BHQ2-3' |

Control reactions were carried out in the presence of an unmodified probe (probes Piv2-Probe1, Piv2-Probe2 and PIV2 TM6). Reactions with template and without template were carried out respectively. The reactions without template were carried out as control for possible occurrence of nonspecific background.

The PCR protocol is 20 min at 50 °C, 5 min at 95 °C, followed by 45 cycles of 15 sec at 95 °C, 45 sec at 60 °C.

Fluorescence data (amplification plots) were registered during the 60 °C step. The RT-PCR analyses were carried out using an RG-Q (Qiagen) with a reaction volume of 25 μl. Subsequently, the PCR products were subjected to a melting profile analysis.

The unmodified probes Piv2-Probe1 and Piv2-Probe1 showed different melting temperatures depending on which template, PIV2.1 or PIV2.2 was present. The melting temperatures of probe PIV2 TM4 C3 modified with the C3-linker did not differ from one another; in the presence of templates carrying a mutation, they did not differ either. In all reactions, the NTCs did not show a peak.

Table 11 (Survey of the melting temperatures of the different probes)

| Probe | Tmelt PIV2.1 (°C) | Tmelt PIV2.2 (°C) | Delta T (Match-Mismatch) (°C) |
|---|---|---|---|
| Piv2-Probe1 | 62,7 (mismatch) | 69 (match) | 6,3 |
| Piv2-Probe2 | 67,95 (match) | 64,95 (mismatch) | 3 |
| PIV2 TM4 C3 (modified) | 62,5 | 62,5 | 0 |

The real-time RT-PCR was repeated with new probes (using unmodified probe PIV2 TM6 and probe PIV2 TM7 C3 modified with C3-linker) which had a modified sequence in comparison to probes Piv2-Probe1, Piv2-TagMelt2 and PIV2 TM4 C3. The results described so far could be confirmed in the real-time RT-PCR as well as in the melting profile analysis. The curves in the real-time RT-PCR can be compared with each other with respect to Ct value and the height of the graph plateau.

This result shows that the probes modified with a C3-linker (probes PIV2 TM4 C3 and PIV2 TM7 C3) are as functional in the real-time RT-PCR as the unmodified probes (probes Piv2-Probe1, Piv2-Probe2 and PIV2 TM6).

The unmodified probe PIV2 TM6 showed different melting temperatures depending on which template, PIV2.1 or PIV2.2, was present in the reaction. The melting temperatures of probe PIV2 TM7 C3 modified with C3-linker did not differ; in the presence of templates carrying a mutation, they did not differ either. In all reactions, the NTCs did not show a peak.

Table 12 (Test of melting temperatures of the different probes)

| Probe | Tmelt PIV2.1 (°C) | Tmelt PIV2.2 (°C) | Delta T (Match-Mismatch) (°C) |
|---|---|---|---|
| PIV2 TM6 | 70,25 (match) | 67,6 (mismatch) | 2,6 |
| PIV2 TM7 C3 (modified) | 65,65 | 65,73 | 0,1 |

As shown in detail above different probes for different target sequences were designed in order to test various aspects of the present invention. Each of the probes used allowed an improvement. In fact, the differences of melting temperature between perfect match and mismatch significantly decreased from 2.6 °C-6.3 °C in the case of a non-modified probe to 0 °C-0.5 °C in the case of a modified probe. The differences of the melting temperature of a modified probe are within the standard deviations of such an experiment.

## Claims

**1.** Method for detecting a nucleic acid, comprising the steps of,

a. amplifying the nucleic acid to be detected,
b. during or after amplification, hybridizing to said nucleic acid to be detected a first probe that comprises an abasic site, additionally carrying a detectable label, wherein the position of the abasic site corresponds to a position known to have a polymorphism in said nucleic acid to be detected,
c. and wherein said nucleic acid is detected if hybridization occurs,

wherein the amplification method is real-time PCR and the melting temperature and/or point of said probe is determined during PCR or post PCR,
wherein the method comprises at least one further probe which also comprises an abasic site but which carries the same label as said first hybridization probe and wherein the probes are distinguishable due to their difference in melting temperatures.

**2.** Method according to claim 1, wherein after hybridization the melting temperature and/or point of said first hybridization probe is determined and the probe carries a label which is detectable when the probe hybridizes to its target or dissociates from its target.

**3.** Method according to claims 1 or 2, wherein the abasic site is derived from a 1'-2'-dideoxyribose or a 1, 3-propandiol.

**4.** Method according to claims 1 to 3, wherein

a. each of the probes is specific for a nucleic acid sequence,
b. at least two probes carry the same label; and
c. each of the probes that carry the same label has a melting temperature ($T_m$) which differs by more than 1 °C from the other probes.

**5.** Method according to claims 1 to 4, wherein the first probe is selected from the group of TaqMan probe, Scorpion probe, molecular beacon probe, light cycler probe, LUX probe and amplifluor probe.

**6.** Oligonucleotide probe selected from the group of TaqMan probe, Scorpion probe, molecular beacon probe, light cycler probe, LUX probe and amplifluor probe, wherein the probe comprises at least one abasic site, and wherein the abasic site corresponds to a position known to have a polymorphism in a nucleic acid to be detected.

**7.** Kit for the detection of a nucleic acid, wherein the kit comprises at least one oligonucleotide probe with an abasic site, and wherein the abasic site corresponds to a position known to have a polymorphism in said nucleic acid to be detected, wherein the probe is labelled and wherein the probe is a TaqMan probe, Scorpion probe, molecular beacon probe, light cycler probe, LUX probe or amplifluor probe.

**8.** Kit according to claim 7, wherein the kit comprises two oligonucleotide probes wherein these two carry the same label, wherein at least one probe carries an abasic site and both are specific for a given target.

## Patentansprüche

**1.** Verfahren zum Nachweis einer Nukleinsäure, umfassend die Schritte:

a. Amplifikation der nachzuweisenden Nukleinsäure,
b. während oder nach der Amplifikation, Hybridisieren der nachzuweisenden Nukleinsäure an einer Sonde die eine abasische Stelle umfasst, die zusätzlich eine nachweisbare Markierung trägt, wobei die Position der abasischen Stelle einer Position entspricht, von der bekannt ist, dass sie einen Polymorphismus in der nachzuweisenden Nukleinsäure aufweist,
c. und wobei die Nukleinsäure detektiert wird, wenn eine Hybridisierung auftritt,

wobei das Amplifikationsverfahren eine real-time PCR ist und die Schmelztemperatur und/oder der Schmelzpunkt der Sonde während der PCR oder nach der PCR bestimmt wird,
wobei das Verfahren mindestens eine weitere Sonde umfasst, die auch eine abasische Stelle umfasst, die aber die gleiche Markierung wie die erste Hybridisierungssonde trägt und wobei die Sonden aufgrund ihrer unterschiedlichen Schmelztemperaturen unterscheidbar sind.

**2.** Verfahren gemäß Anspruch 1, wobei nach der Hybridisierung die Schmelztemperatur und/oder der Schmelzpunkt der ersten Hybridisierungssonde bestimmt wird und die Sonde eine Markierung trägt, die nachweisbar ist, wenn die Sonde an ihr Ziel hybridisiert oder von ihrem Ziel dissoziiert.

**3.** Verfahren gemäß Anspruch 1 oder 2, wobei die abasische Stelle von einer 1'-2'-Didesoxyribose oder einem 1,3-Propandiol abstammt.

**4.** Verfahren gemäß den Ansprüchen 1 bis 3, wobei

a. jede der Sonden spezifisch für eine Nukleinsäuresequenz ist,
b. mindestens zwei Sonden die gleiche Markierung tragen; und
c. jede der Sonden, die die gleiche Markierung tragen, eine Schmelztemperatur (Tm), hat die um mehr als 1°

C von den anderen Sonden abweicht.

**5.** Verfahren gemäß den Ansprüchen 1 bis 4, wobei die erste Sonde ausgewählt ist aus der Gruppe von TaqMan-Sonde, Scorpion-Sonde, Molecular Beacon-Sonde, Lichtcycler-Sonde, LUX-Sonde und Amplifluor-Sonde.

**6.** Oligonukleotidsonde, ausgewählt aus der Gruppe von TaqMan-Sonde, Scorpion-Sonde, Molecular-Beacon-Sonde, Lichtcycler-Sonde, LUX-Sonde und Amplifluor-Sonde, wobei die Sonde mindestens eine abasische Stelle umfasst und wobei die abasische Stelle einer Position entspricht, von der bekannt ist, dass sie einen Polymorphismus in der nachzuweisenden Nukleinsäure aufweist.

**7.** Kit zum Nachweis einer Nukleinsäure, wobei das Kit mindestens eine Oligonukleotidsonde mit einer abasischen Stelle umfasst und wobei die abasische Stelle einer Position entspricht, von der bekannt ist, dass sie einen Polymorphismus in der nachzuweisenden Nukleinsäure aufweist, wobei die Sonde markiert ist und wobei die Sonde eine TaqMan-Sonde, Scorpion-Sonde, Molecular Beacon-Sonde, Lichtcycler-Sonde, LUX-Sonde oder Amplifluor-Sonde ist.

**8.** Kit gemäß Anspruch 7, wobei das Kit zwei Oligonukleotidsonden umfasst, wobei diese zwei die gleiche Markierung tragen, wobei mindestens eine Sonde eine abasische Stelle trägt und beide spezifisch für ein gegebenes Ziel sind.

## Revendications

**1.** Procédé destiné à détecter un acide nucléique, comprenant les étapes consistant à,

a. amplifier l'acide nucléique devant être détecté,
b. pendant ou après l'amplification, hybrider avec ledit acide nucléique devant être détecté une première sonde qui comprend un site abasique, portant en plus un marqueur détectable, dans laquelle la position du site abasique correspond à une position connue pour avoir un polymorphisme dans ledit acide nucléique devant être détecté,
c. et dans lequel ledit acide nucléique est détecté si l'hybridation a lieu,

dans lequel le procédé d'amplification est une ACP en temps réel et la température et/ou le point de fusion de ladite sonde est déterminé(e) pendant l'ACP ou après l'ACP,
ledit procédé comprenant au moins une sonde supplémentaire qui comprend aussi un site abasique mais qui porte le même marqueur que ladite première sonde d'hybridation, et dans lequel les sondes sont distinguables de par leur différence de températures de fusion.

**2.** Procédé selon la revendication 1, dans lequel, après l'hybridation, la température et/ou le point de fusion de ladite première sonde d'hybridation est déterminé(e) et la sonde porte un marqueur qui est détectable quand la sonde s'hybride avec sa cible ou se dissocie de sa cible.

**3.** Procédé selon les revendications 1 ou 2, dans lequel le site abasique est dérivé d'un 1'-2'-didésoxyribose ou d'un 1,3-propanediol.

**4.** Procédé selon les revendications 1 à 3, dans lequel

a. chacune des sondes est spécifique d'une séquence d'acide nucléique,
b. au moins deux sondes portent le même marqueur ; et
c. chacune des sondes qui portent le même marqueur a une température de fusion ($T_m$) qui diffère de plus de 1°C des autres sondes.

**5.** Procédé selon les revendications 1 à 4, dans lequel la première sonde est choisie dans le groupe des sonde TaqMan, sonde Scorpion, sonde balise moléculaire, sonde "light cycler", sonde LUX et sonde amplifluor.

**6.** Sonde d'oligonucléotides choisie dans le groupe des sonde TaqMan, sonde Scorpion, sonde balise moléculaire, sonde "light cycler", sonde LUX et sonde amplifluor, la sonde comprenant au moins un site abasique, et dans laquelle le site abasique correspond à une position connue pour avoir un polymorphisme dans un acide nucléique devant être détecté.

**7.** Trousse de détection d'un acide nucléique, la trousse comprenant au moins une sonde d'oligonucléotides avec un site abasique, et dans laquelle le site abasique correspond à une position connue pour avoir un polymorphisme dans ledit acide nucléique devant être détecté, dans laquelle la sonde est marquée et dans laquelle la sonde est une sonde TaqMan, une sonde Scorpion, une sonde balise moléculaire, une sonde "light cycler", une sonde LUX ou une sonde amplifluor.

**8.** Trousse selon la revendication 7, la trousse comprenant deux sondes d'oligonucléotides, ces deux-là portant le même marqueur, dans laquelle au moins une sonde porte un site abasique et les deux sont spécifiques d'une cible donnée.

**FIG. 1**

**C3 Linker**

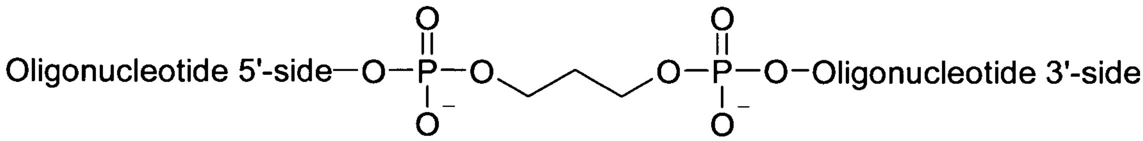

**dSpacer**

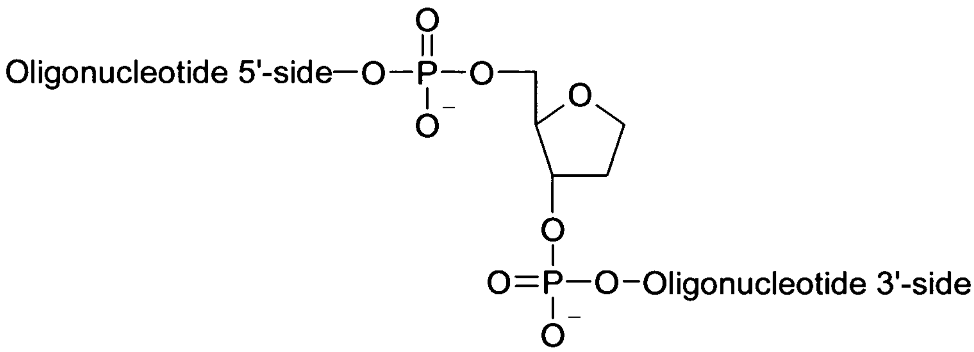

**FIG. 2**

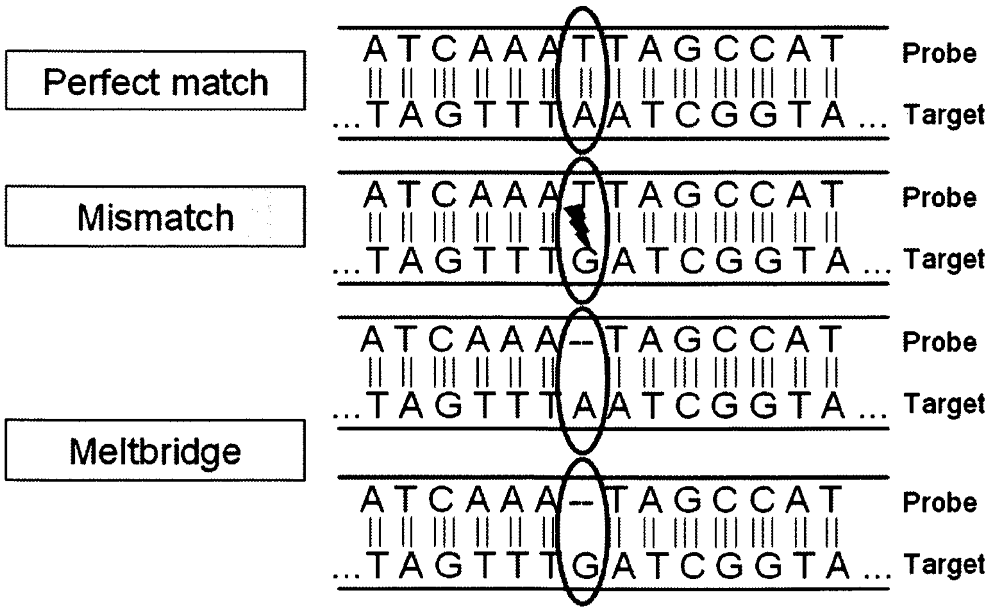

FIG. 3

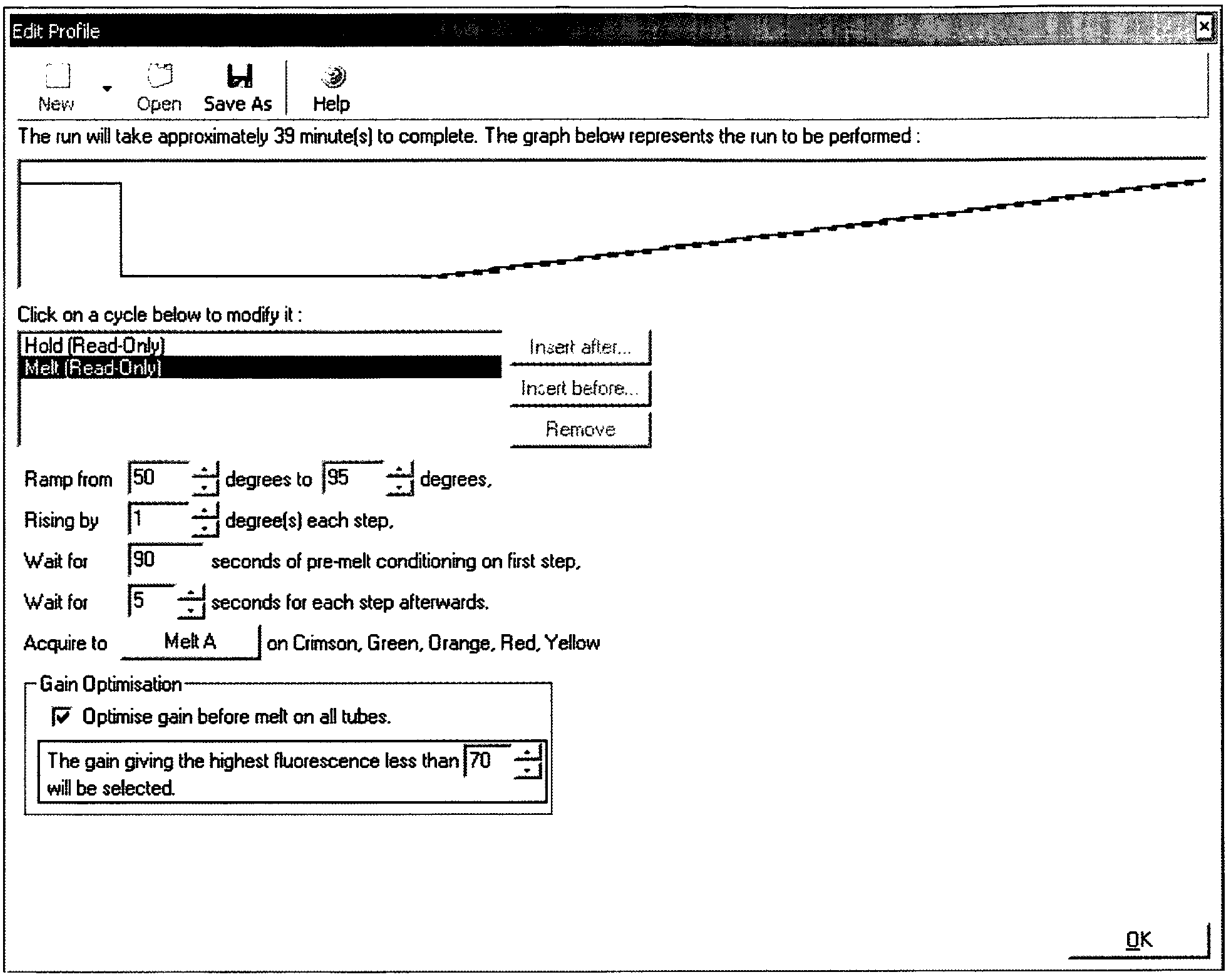
Edit Profile
New
Open
Save As
Help
The run will take approximately 39 minute(s) to complete. The graph below represents the run to be performed :
Click on a cycle below to modify it :
Hold (Read-Only)
Melt (Read-Only)
Insert after...
Insert before...
Remove
Ramp from 50 degrees to 95 degrees,
Rising by 1 degree(s) each step,
Wait for 90 seconds of pre-melt conditioning on first step,
Wait for 5 seconds for each step afterwards.
Acquire to Melt A on Crimson, Green, Orange, Red, Yellow
Gain Optimisation
Optimise gain before melt on all tubes.
The gain giving the highest fluorescence less than 70 will be selected.
OK

FIG. 4

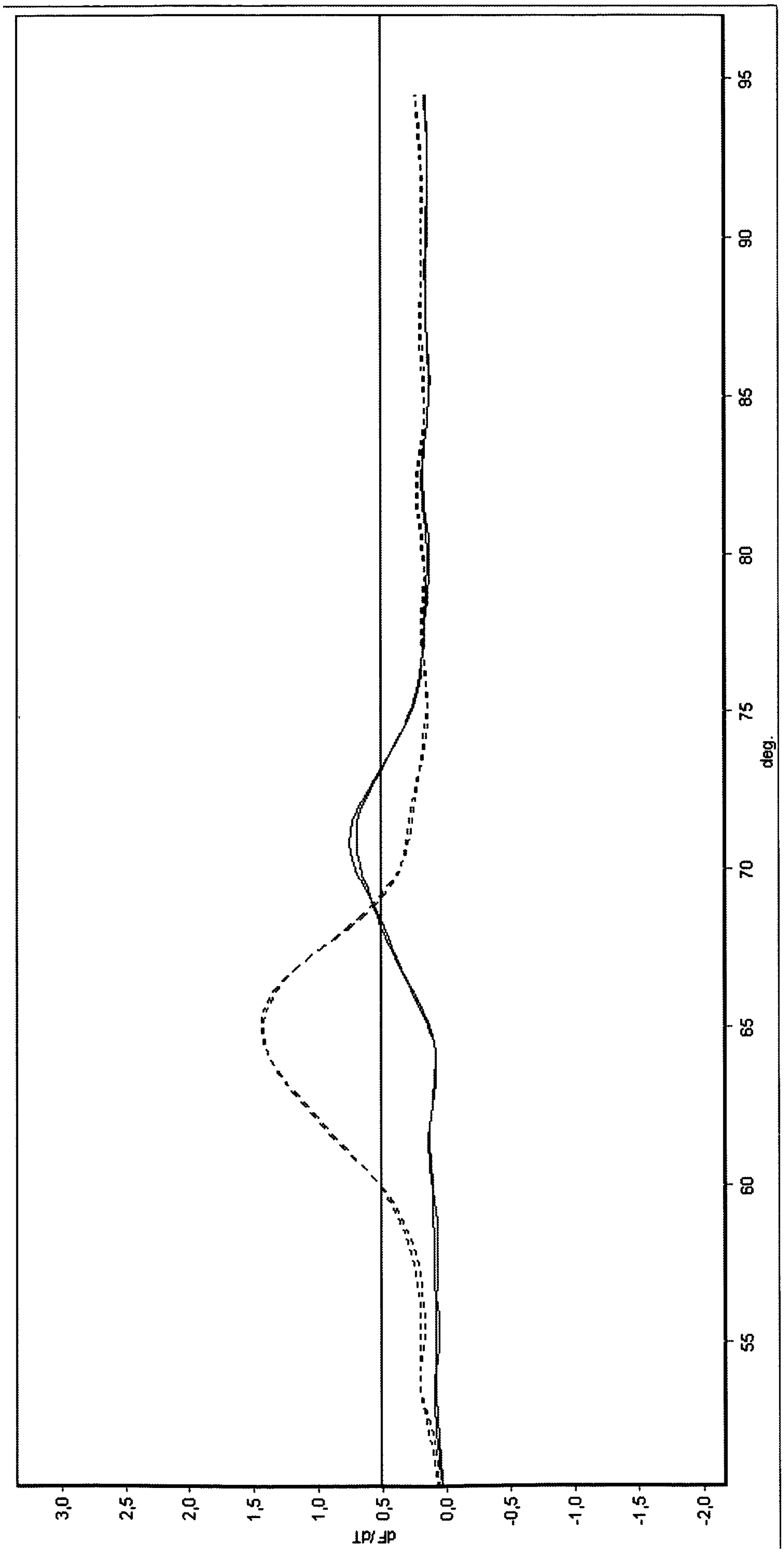

FIG. 5

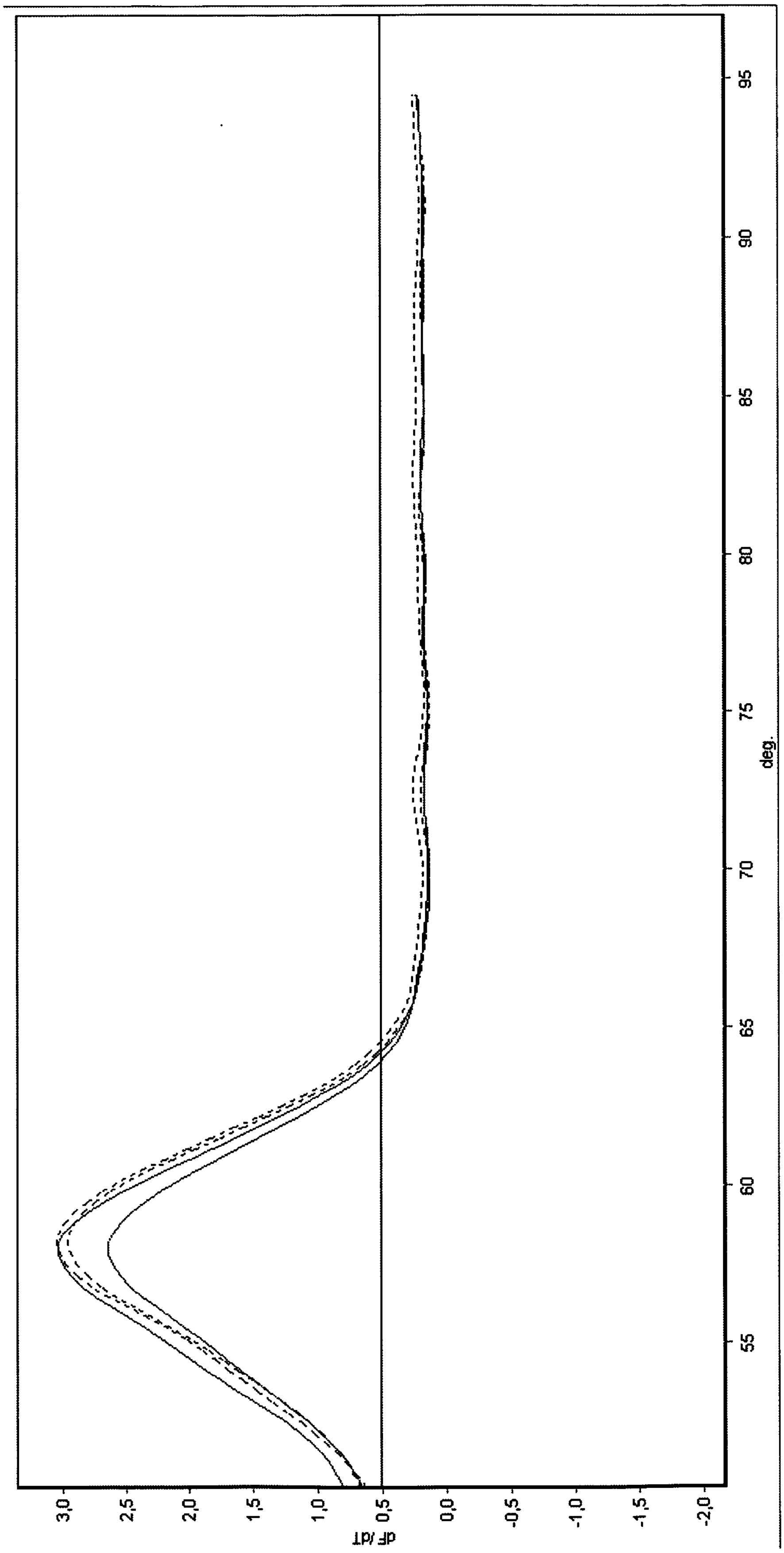

FIG. 6

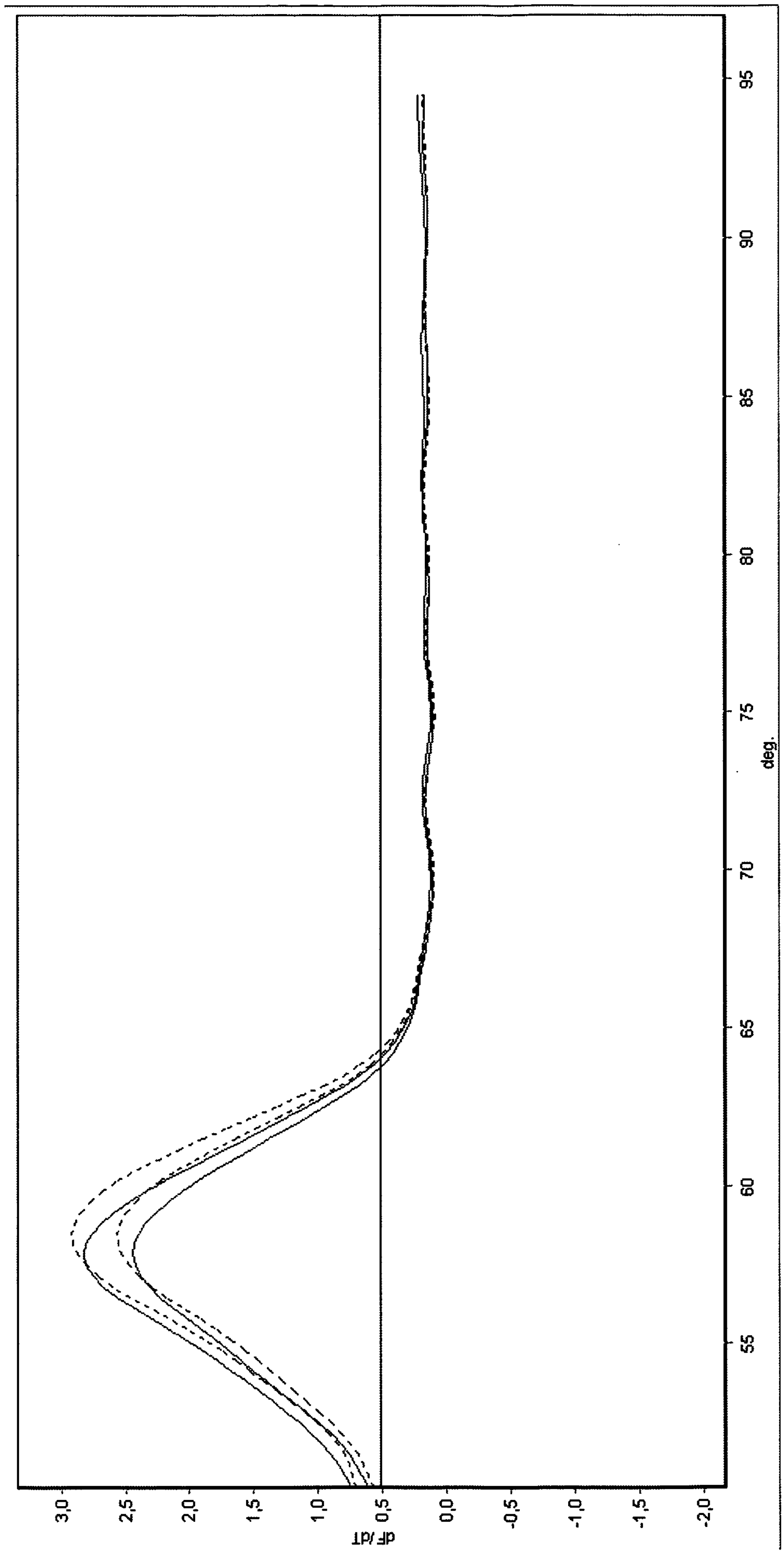

FIG. 7

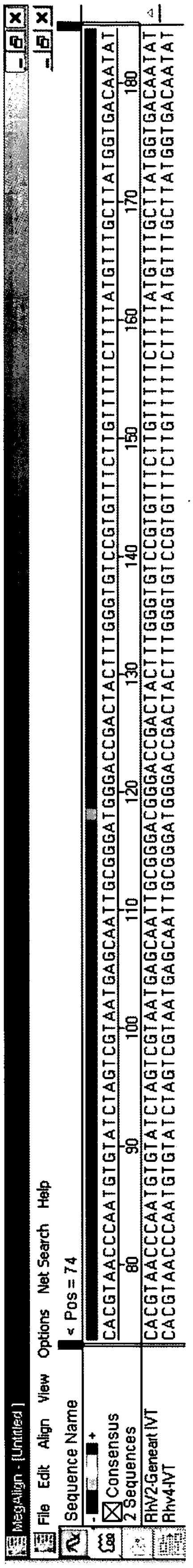
MegAlign - [Untitled]
File Edit Align View Options Net Search Help
Sequence Name
Pos = 74
Consensus
2 Sequences
RhV2-Geneart IVT
Rhv4-IVT

FIG. 8

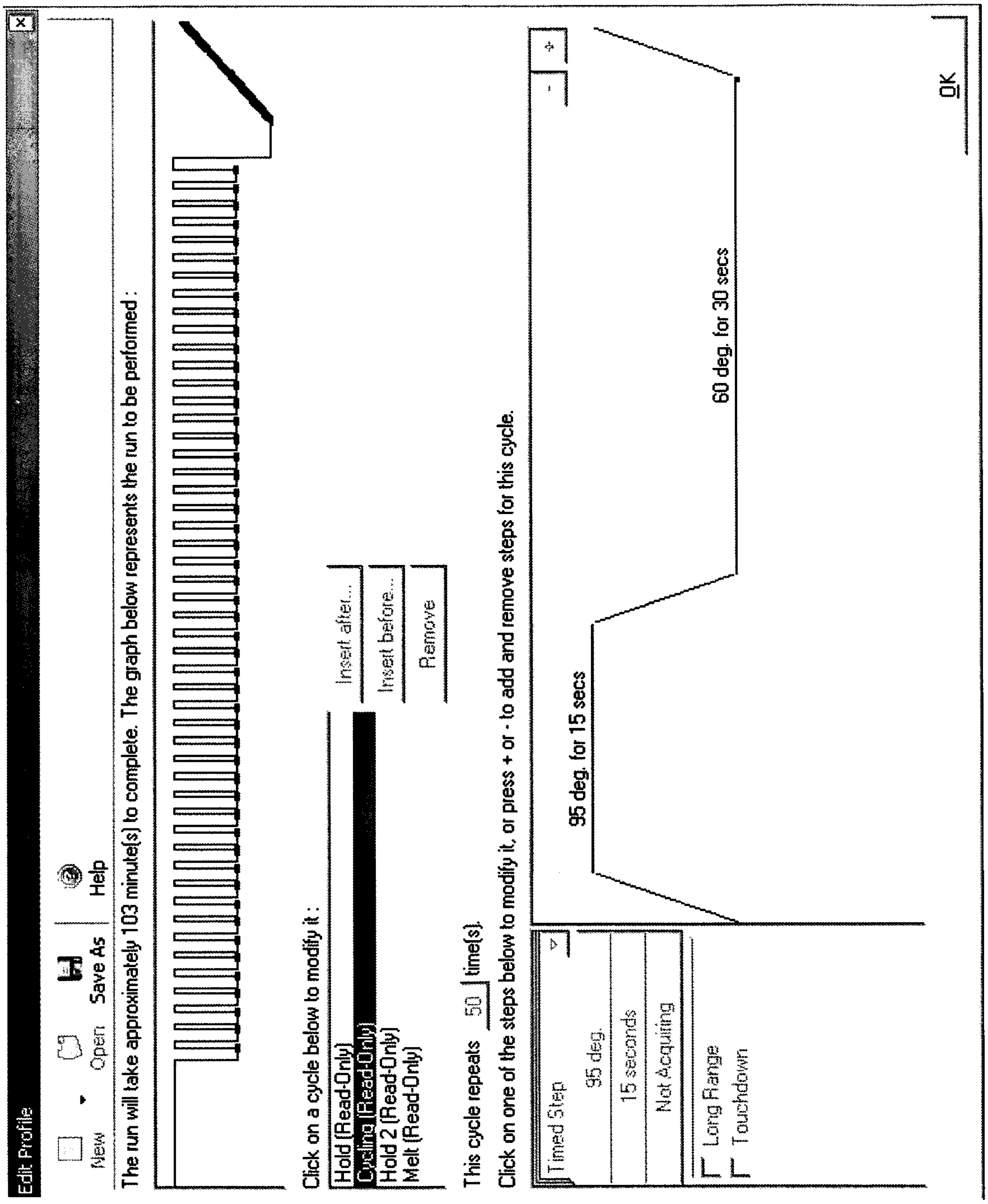
Edit Profile
New
Open
Save As
Help
The run will take approximately 103 minute(s) to complete. The graph below represents the run to be performed :
Click on a cycle below to modify it :
Hold (Read-Only)
Cycling (Read-Only)
Hold 2 (Read-Only)
Melt (Read-Only)
Insert after...
Insert before...
Remove
This cycle repeats 50 time(s).
Click on one of the steps below to modify it, or press + or - to add and remove steps for this cycle.
Timed Step
95 deg.
15 seconds
Not Acquiring
Long Range
Touchdown
95 deg. for 15 secs
60 deg. for 30 secs
OK

FIG. 9

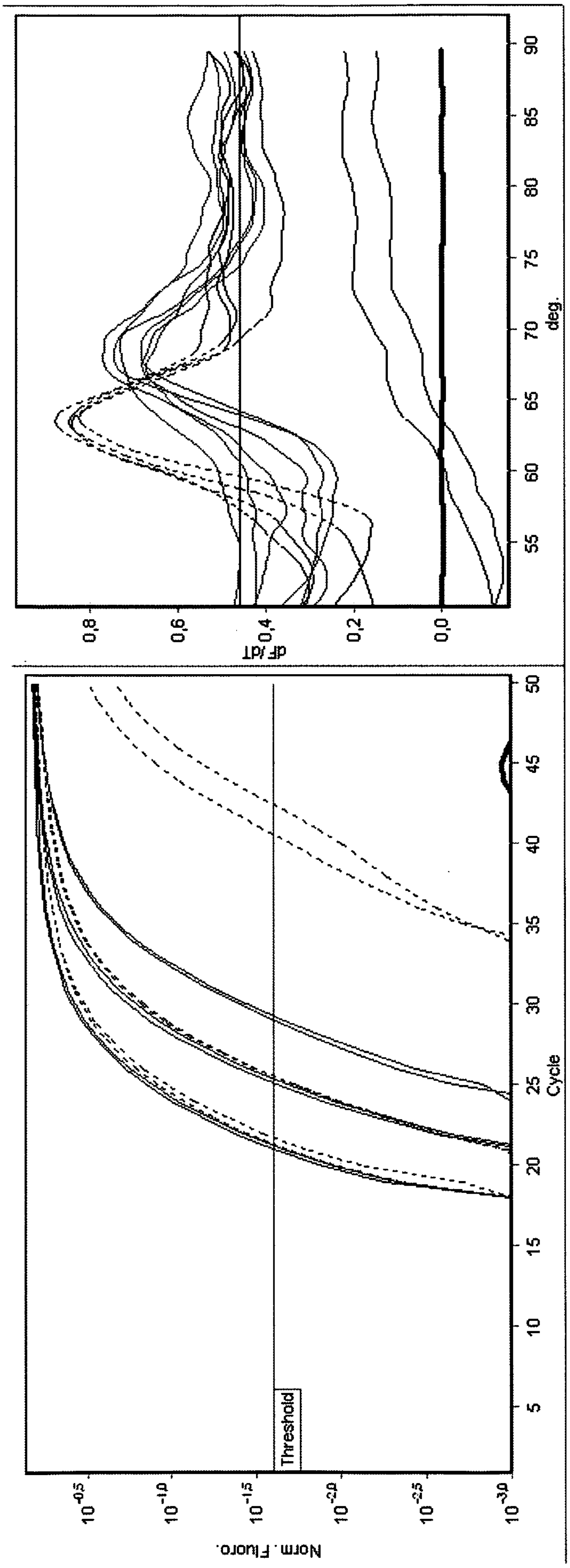

FIG. 10

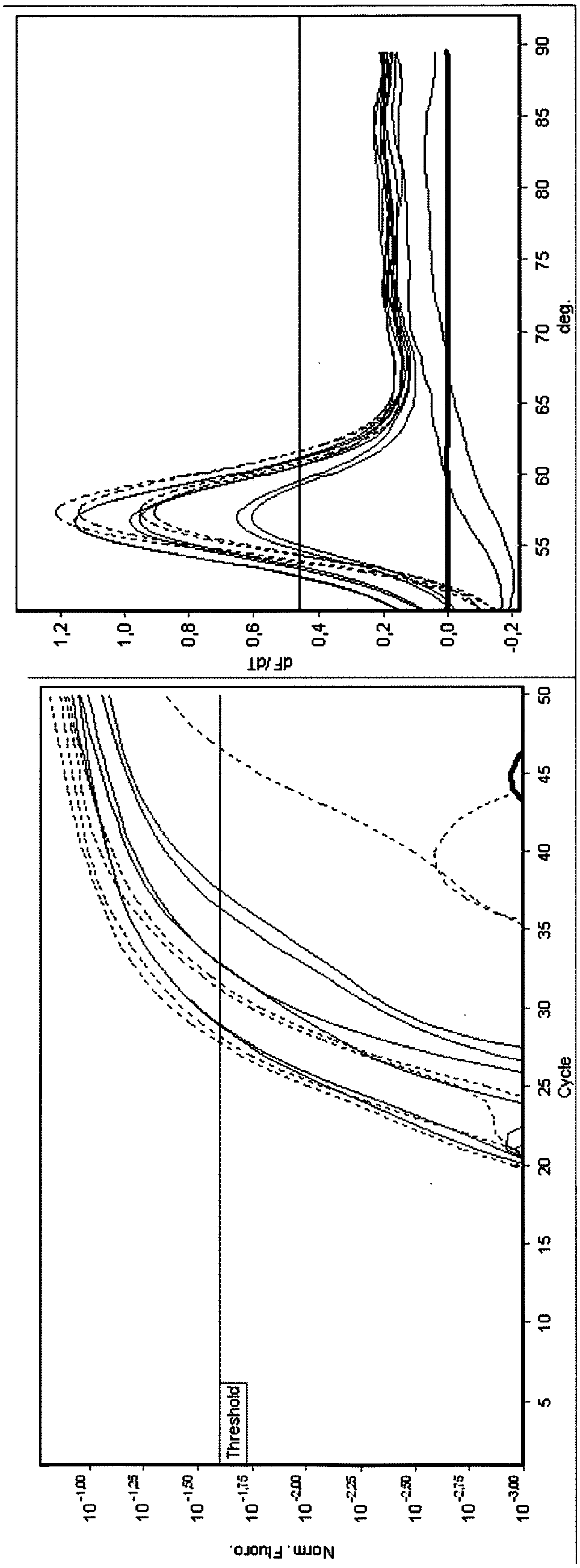
Norm. Fluoro.
10^-1.00
10^-1.25
10^-1.50
10^-1.75
10^-2.00
10^-2.25
10^-2.50
10^-2.75
10^-3.00
Threshold
5
10
15
20
25
30
35
40
45
50
Cycle
dF/dT
1,2
1,0
0,8
0,6
0,4
0,2
0,0
-0,2
55
60
65
70
75
80
85
90
deg.

FIG. 11

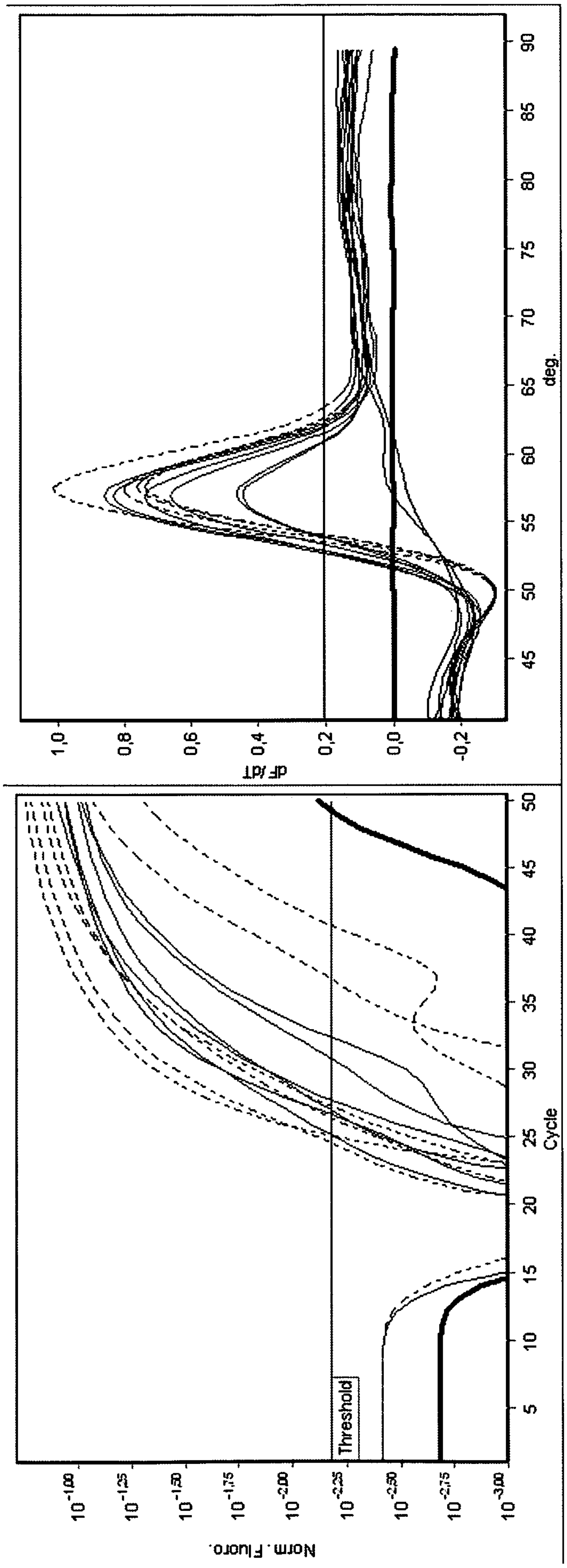

Norm. Fluoro.
$10^{-1.00}$
$10^{-1.25}$
$10^{-1.50}$
$10^{-1.75}$
$10^{-2.00}$
$10^{-2.25}$
$10^{-2.50}$
$10^{-2.75}$
$10^{-3.00}$
Threshold
5
10
15
20
25
30
35
40
45
50
Cycle
dF/dT
1,0
0,8
0,6
0,4
0,2
0,0
-0,2
45
50
55
60
65
70
75
80
85
90
deg.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009135832 A
- US 6361940 B1
- US 6579680 B1
- US 4458006 A
- EP 11159652 A


### Non-patent literature cited in the description


- **MELCHIOR ; VON HIPPEL.** *Proc. Nat. Acad. Sci. USA,* 1973, vol. 70 (2), 298-302
- **CHEVET ; LEMAITRE ; KATINKA.** *Nucleic Acids Research,* 1995, vol. 23 (16), 3343-3344
- **WATKINS, JR ; SANTALUCIA, JR.** *Nucleic Acids Research,* 2005, vol. 33 (19), 6258-6267
- **LIU et al.** Rolling circle DNA synthesis: Small circular oligonucleotides as efficient templates for DNA polymerases. *J. Am. Chem. Soc.,* 1996, vol. 118, 1587-1594
- **WALKER et al.** Strand displacement amplification an isothermal, in vitro DNA amplification technique. *Nucleic Acids Res.,* 1992, vol. 20 (7), 1691-6
- **LANDEGREN et al.** A Ligase-Mediated Gene Detection Technique. *Science,* 1988, vol. 241, 1077-1080
- Ligase Chain Reaction (LCR)--Overview and Applications. **WIEDMANN et al.** PCR Methods and Applications. Cold Spring Harbor Laboratory Press, 1994, S51-S64
- **BEAUCAGE et al.** *Tetrahedron Letters,* 1981, vol. 22, 1859-1862
- **MANIATIS et al.** Molecular Cloning: A Laboratory Manual. Cold Spring Harbor Laboratory, 1982, 280-281